# EUROPEAN PATENT SPECIFICATION

(11) **EP 1 005 272 B1**
(45) Date of publication and mention of the grant of the patent: **23.07.2003**
(21) Application number: 98955395.3
(22) Date of filing: 18.08.1998
(51) Int. Cl.: A01N 47/24

(54) **Method of increasing foreign protein expression**
Verfahren zur Steigerung der Expression von Fremdproteinen
Procédé permettant d'augmenter l'expression des protéines étrangères

(30) Priority: 22.08.1997 US 56638 P; 27.03.1998 US 79628 P; 27.03.1998 FR 9804067; 06.05.1998 US 84477 P
(43) Date of publication of application: 07.06.2000
(73) Proprietor: Bayer CropScience S.A., 69009 Lyon (FR)
(72) Inventor: HOLMES, Keith, A., Cary, NC 27511 (US); STAHL, Daniel, D., Raleigh, NC 27612 (US)
(86) International application number: EP9805701
(87) International publication number: WO99009830

(56) References cited:
- C. TOMLIN (ED): "The Pesticide Manual" , BRITISH CROP PROTECTION COUNCIL , UK XP002091614 10th edition,1994 see page 24 - page 26

## Description

The present invention relates to a method of increasing foreign protein expression in plants, a method of controlling insects at a locus, a method of increasing the time during which an herbicide may be applied to a herbicide tolerant plant, a plant in which the expression of a foreign protein has been increased by the application of aldicarb and a method of growing crops.

It is known in the field of plant biotechnology that heterologous genes encoding foreign proteins which impart herbicide tolerance, disease resistance or toxicity to insects (insect resistance), or improve the quality of the crops, can be incorporated into the genome of certain plants. These genetically transformed plants can express these proteins for the added protection of the plants, or higher quality of the crops. Genes coding for protein imparting herbicide tolerance are known in the art, including genes imparting tolerance to oxynil herbicides (US 4,810,648 and US 5,559,024), genes imparting tolerance to glyphosate and EPSPS inhibitor herbicides (US 4,535,060, US 4,769,061, US 5,094,945, US 4,940,835, US 5,188,642, US 4,971,908, US 5,145,783, US 5,312,910, US 5,310,667, US 5,633,435, US 5,627,061, US 5,554,798, US 5,633,448, WO 97/04103), genes imparting tolerance to glufosinate (EP 242 236), as well as genes imparting tolerance to HPPD inhibitors (WO 96/38567 and WO 98/02562). Genes coding for proteins imparting disease resistance are known in the art, including lytic peptides, oxalate oxydase genes for tolerance to sclerotinia or chitinases. Genes imparting insect resistance are also known in the art, including genes which encode for *Bacillus thuringiensis* (*Bt*) endotoxins (Inter alia, 5,460,963, 5,683,691, 5,545,565, 5,530,197, 5,317,096) etc., or for insecticidal toxins isolated from *Photorhabdus* (WO 97/17432 or WO 98/08932). In general, these heterologous genes have been placed in a variety of plants including cotton.

It is also known that the levels of protein encoded by the heterologous genes in the transgenic plants can vary according to *inter alia* environmental conditions. For example, the grower of the said plants can be led to believe that the said plants are capable of tolerance to herbicide without being substantially damaged by the herbicide, or capable of resistance to diseases or withstanding attack by insect pests without further assistance from other pesticides. However, it is a severe problem for the grower if the said plants do not express the said protein at the correct time, and in an effective amount, particularly a pesticidally effective amount, more particularly an insecticidally effective amount in case of insect resistance. For example, in the case of *Bt* endotoxin, if there is not enough in the said plants, then the plants are susceptible to damage from pests, particularly lepidopteran pests, including bollworms. Cotton is such a plant that may be severely affected by bollworm attack. For another example, in the case of cotton seeds or plants comprising a gene encoding a Type II EPSPS, like Roundup Ready® Cotton, the window for application of EPSPS inhibitor herbicides is very strict and narrow (before the 4 leaves stage), and bolls may be damaged when the level of EPSPS expression is not sufficient to impart a proper level of tolerance to glyphosate, leading to an unacceptable loss of yield for the farmer.

Although it is known that aldicarb is a systemically acting insecticide, acaricide and nematicide and generally provides plants with a plant growth promoting effect, aldicarb is not generally effective against lepidopteran species when applied at the seed. Aldicarb is also known as 2-methyl-2-(methylthio)propionaldehyde O-(methylcarbamoyl)oxime.

An object of the present invention is to enhance the expression of heterologous proteins encoded by genes in plants.

Another object of the present invention is to enhance the expression of *Bacillus thuringiensis* endotoxin in plants.

Another object of the present invention is to enhance the expression of proteins imparting herbicide tolerance in plants.

Another object of the present invention is to provide an improved method for the control of pests at a locus.

Another object of the present invention is to provide an improved method of application of an herbicide to a plant tolerant to the said herbicide.

Another object of the invention is to provide an improved plant protected from predacious insects.

Another object of the invention is to provide a plant with improved protection from herbivorous insects.

Another object of the invention is to provide an improved plant comprising a foreign gene which encodes for a foreign protein.

Another object of the invention is to provide an improved seed comprising a foreign gene which encodes for a foreign protein.

These objects are met in whole or in part by the present invention.

The present invention relates to a method of increasing foreign protein expression encoded by a foreign gene in plants comprising treating the plant or a seed from which it grows with aldicarb.

By the term "heterologous gene" or "foreign gene" is meant a gene that has been transformed or introduced into the plant, particularly integrated into the transformed plant genome, for example, transformed into the plant or an ancestor thereof, more particularly a gene that is not naturally present in the transformed plant, or even more particularly a gene which contains a sequence coding for a heterologous or foreign protein, including regulatory elements appropriate for controlling the expression of the said coding sequence in the plant cells, for example, promoters, terminators, pre-pro peptides, or transit peptides, the latter driving the expression of the said heterologous protein in a specific targeted region of the plant cell.

Promoters controlling the expression of a gene in plant cells are well known in the field of plant biotechnology, including any promoter sequence of a gene naturally expressed in plants or plant cells, from plant, viral or bacterial origin. Suitable such promoters are disclosed in Weising & al (1988, Annual Rev. Genet., 22:241). The following is a partial representative list of promoters suitable for use herein : regulatory sequences from the T-DNA of *A. tumefaciens,* including mannopine synthase, nopaline synthase and octopine synthase ; regulatory sequences from plant origin, including alcohol deshydrogenase promoter from corn, light inducible promoters such as ribulose-biscarboxylase/oxygenase small subunit promoters (SSU RuBisCO) from genes of a variety of species and the major chlorophyl a/b binding gene promoters, histone promoters (EP 507 698), actin promoters, maize ubiquitin 1 promoters (Christenses & al., 1996, *Transgenic Res*., 5 :213); regulatory sequences from viral origins, such as 19S or 35S promoters of the cauliflower mosaic virus, (US 5,352,605 ; US 5,530,196) ; developmentally regulated promoters such as waxy, zein, or bronze peomoters from maize; as well as synthetic or other natural promoters which are either inducible or constitutive, including those promoters exhibiting organ specific expression or expression at specific development stage(s) of the plant, like the promoter of napin (EP 255 378) or the alpha-tubulin promoter (US 5,635,618).

By the term "heterologous protein" or "foreign protein" is meant a protein of interest produced by the expression of the foreign gene in the plants. It may be a protein naturally present in the transformed plant specie but expressed at levels or in specific plant tissues or targeted regions of the plant cells, not naturally occurring in the said transformed plant species, or a protein not naturally present in the transformed plant specie.

The invention provides a method of increasing foreign gene expression in transgenic plants which method comprises treating the plant or a seed from which it grows with aldicarb.

The transgenic plants according to the invention include cotton, corn, potato, soybean, sunflower, rape seed oil, sugar cane, sugar beet, rice, alfalfa, or banana plants, preferably cotton, corn or potato plants, most preferably a cotton plant.

Preferably, the foreign genes according to the present invention are all the foreign or heterologous genes coding for proteins of interest to impart herbicide tolerance , disease resistance or toxicity to insects (insect resistance), or to improve quality of the crops, known in the art and/or disclosed here above.

As a preferred embodiment of the present invention, the heterologous or foreign gene encodes a protein to impart herbicide tolerance, more preferably tolerance to an oxynil herbicide (disclosed in US 4,810,648 and US 5,559,024), to EPSPS inhibitor herbicides, including glyphosate and its various salts (disclosed in US 4,535,060, US 4,769,061, US 5,094,945, US 4,940,835, US 5,188,642, US 4,971,908, US 5,145,783, US 5,312,910, US 5,310,667, US 5,633,435, US 5,627,061, US 5,554,798, US 5,633,448, WO 97/04103), to glufosinate (EP 242 236), or to HPPD inhibitors (WO 96/38567 and WO 98/02562). More preferably, the heterologous or foreign gene encodes a protein to impart tolerance to EPSPS inhibitor herbicides.

As another preferred embodiment of the present invention, the heterologous or foreign gene encodes a protein to impart insect resistance, more preferably genes which encode for *Bacillus thuringiensis* (*Bt*) endotoxins (Inter alia, US 5,460,963, US 5,683,691, US 5,545,565, US 5,530,197, US 5,317,096).

The genes that are preferably embraced by the instant invention are *cryI, cryII, cryIII,* and *cryIV* genes. More preferably, the genes include: *cryIA(a); cryIA(b); cryIA(c);* and *cryIIIA(a).* Most preferably the gene is *cryIA(a), cryIA(b)* or *cryIA(c).* The type of plant which expresses a *Bt* endotoxin is known as a "Bt" plant, for example "Bt-cotton", "Bt-corn" or "Bt-potato."

The promoters controlling the expression of the above genes in plant cells include the following : regulatory sequences from the T-DNA of *A. tumefaciens,* including mannopine synthase, nopaline synthase and octopine synthase; regulatory sequences from plant origin, including alcohol deshydrogenase promoter from corn, light inducible promoters such as ribulose-biscarboxylase/oxygenase small subunit promoters (SSU RuBisCO) from genes of a variety of species and the major chlorophyl a/b binding gene promoters, histone promoters (EP 507 698), actin promoters, maize ubiquitin 1 promoters (Christensen & al., 1996, *Transgenic Res*., 5 :213) ; regulatory sequences from viral origins, such as 19S or 35S promoters of the cauliflower mosaic virus, (US 5,352,605 ; US 5,530,196); developmentally regulated promoters such as waxy, zein, or bronze promoters from maize ; as well as synthetic or other natural promoters which are either inducible or constitutive, including those promoters exhibiting organ specific expression or expression at specific development stage(s) of the plant, like the napin promoter (EP 255 378) or the alpha-tubulin promoter (US 5,635,618). As a preferred embodiment, the promoter is selected among the group consisting in the ribulose-biscarboxylase/oxygenase small subunit promoters (SSU RuBisCO) from genes of a variety of species , the histone promoters, the actin promoters, the maize ubiquitin 1 promoters and the 35S promoters of the cauliflower mosaic virus (CaMV 35S), most preferably the CaMV 35 S promoter.

The transgenic plant may be a *Bt*-plant in which the foreign gene expresses *Bt*-endotoxin and/or an herbicide tolerant plant in which the foreign gene expresses a foreign protein imparting tolerance to the said herbicide.

Preferably the transgenic plants are cotton plants, most preferably NuCotn® 32B or NuCotn® 33B or those comprising a foreign gene which encode the *cryIA(a)* or *cryIA(c)* genes, BXN® Cotton comprising a foreign gene which encodes a nitrilase for oxynil tolerance, or Roundup Ready® Cotton comprising a foreign gene which encodes for an EPSPS for glyphosate tolerance.

The foreign gene expression can be elevated in any part of the plant, depending upon the result to be achieved. For herbicide tolerance, the foreign gene should be expressed at high levels in all the parts of the plants, more particularly in the green tissues of the transformed plants, e.g., the leaves and reproductive structures. For insecticidal activity, particularly *Bt,* or herbicide tolerance, the foreign gene may be expressed preferably in the fruiting parts of the cotton plant, most preferably the squares or bolls and may depend on the exact variety of the plant.

Aldicarb may be applied directly to the plant and/or to the seed and/or to a locus where the plant is growing or is to be grown. By the term "to the seed" is meant on or near the seed.

In one embodiment, aldicarb may be applied to the seed in a seed treatment step before or just after planting. That is, the aldicarb and the seed may be placed in the ground simultaneously or sequentially.

Aldicarb may be applied to the locus before or after planting. The plants may be treated with aldicarb by known methods of application such as side-dressing or in-furrow application of granules or liquid; or by drilling.

In one other embodiment, aldicarb may be applied to the locus after planting but before emergence of the plant.

In a preferred aspect of the invention, the seeds are treated after planting with aldicarb at a rate of from 0.01 to 6 kg/ha (kilograms of active ingredient per hectare) preferably from 0.05 to 4 kg/ha more preferably from 0.1 to 1 kg/ha. Generally the amount of aldicarb per 100 meters of crop row is of from 10 to 500 g/100m (grams per 100 meters), preferably from 20 to 100 g/100m.

In another preferred embodiment of the invention, the seeds after treatment with aldicarb before planting may comprise from 0.001 % w/w to 20% w/w of aldicarb to seed, preferably from 0.05% to 10%, more preferably from 0.1% to 5%.

The present invention also relates to a method of controlling pests at a locus, which locus comprises a plant containing a gene which encodes for *Bt* endotoxin, the method comprising the application of aldicarb to the plant or to the seed from which it grows.

It has been unexpectedly found that the application of aldicarb to the plant or to the seed from which it grows improves the protection of the plant from attack by pests, preferably insect pests, more preferably of the Orders Lepidoptera, Coleoptera or Diptera. More preferably, the pests are of the family Noctuidae, even more preferably *Heliothis* spp. or *Helicoverpa* spp. Another pest species controlled by the method of the instant invention is *Pectinophora spp* particularly *Pectinophora gossypiella.* Specific pests controlled by the instant invention include at least one of the following species: *Helicoverpa armigera* (also known as *Heliothis armigera*)*; Helicoverpa virescens* (also known as *Heliothis virescens* or Tobacco budworm); *Heliothis punctigera; Heliothis zea (*cotton bollworm*); Heliothis assulta; and Heliothis peltigera.*

Other Lepidoptera that may be controlled by the method of the present invention include *Ostrinia nubilalis, Spodoptera littoralis, Spodoptera exigua, Spodoptera frugiperda, Manduca sexta, Pieris rapae, Trichoplusia ni,* and *Alabama argillacea.*

Coleoptera that may be controlled by the method of the present invention include the family Crysomelidae, particularly *Leptinotarsa decemlineata* and *Diabrotica undecimpunctata howardi.*

Diptera that may be controlled by the method of the present invention include insects of the family Phorbia, particularly *Phorbia brassicae* and *Phorbia platura.*

The transgenic Bt-plants according to the invention include cotton, corn, potato, sugar cane or rice plants, preferably cotton, corn or potato plants, most preferably a cotton plant.

In a highly preferred aspect of the instant invention, it has been unexpectedly found that in Bt-cotton plants treated with aldicarb, the control of certain species of lepidoptera, most preferably Heliothis armigera, is higher than in those Bt-cotton plants not treated with aldicarb. The *Bt*-cotton in the instant invention is most commonly known commercially as NuCotn 32B or NuCotn 33B or Ingard.

The present invention also relates to a method of controlling weeds at a locus, which locus comprises a plant containing a foreign gene which encodes a protein imparting tolerance to an herbicide, wherein the said herbicide is applied in said locus to said plant, the method comprising the application of aldicarb to the plant or to the seed from which it grows.

The present invention also relates to a method of increasing the time during which an herbicide may be applied to an herbicide tolerant plant, which method comprises applying aldicarb to the said plant or to a seed from which it grows.

As a preferred embodiment, the herbicide is an EPSPS inhibitor herbicide, including glyphosate and its various salts, like the isopropylammonium salt or the sulfosate salt (i.e. Roundup® or Touch Down ®), and the foreign gene encodes for a protein imparting tolerance to the said EPSPS inhibitor herbicide.

It has been unexpectedly found that the application of aldicarb, not only increases the tolerance to the herbicide of the transgenic plant, but also increase the time during which the glyphosate may be applied in said locus to said plant (glyphosate being understood as the representative of the whole familly of EPSPS inhibitor herbicides).

The transgenic herbicide tolerant according to the invention include cotton, corn, soybean, sunflower, rape seed oil, sugar cane, sugar beet or alfalfa plants, preferably cotton, corn or soybean plants, most preferably a cotton plant.

The number of applications of EPSPS inhibitor herbicides, including glyphosate or a salt thereof, per growing season may also be increased, in general from 1 to 7 sprays per season, preferably 2 to 6 sprays per season, more preferably 2 to 5 sprays per season.

In the case of cotton, glyphosate or a salt thereof may be applied on the plant from the emergence of the plant to harvest of the cotton, preferably from emergence to boll opening, more preferably from emergence to the 12 leaf stage, even more preferably from emergence to the 8 leaf stage, even more preferably from emergence to the 6 leaf stage.

The present invention also relates to a new composition which comprises aldicarb and a seed containing a foreign gene which encodes for a foreign protein. The foreign gene and the seeds of the plant may be one of those disclosed above. The composition may comprise from 0.001 % w/w to 20% w/w of aldicarb to seed, preferably from 0.05% to 10%, more preferably from 0.1% to 5%. The aldicarb may coat the seed in a conventional manner as known to the skilled addressee in the art of seed coatings. In addition, the aldicarb may be injected into the seed before planting.

The present invention also relates to a plant which comprises a foreign gene which encodes for a foreign protein as disclosed here above which has been treated by aldicarb, the said plant having an improved amount of foreign protein.

In the case of insect tolerance and *Bt* endotoxin, the plant preferably has more endotoxin in the fruiting parts of the plant than in the leaves; in the case of cotton, in the bolls and squares than in the leaves.

Aldicarb, its uses and formulations are described inter alia in The Pesticide Manual, 10^{th} edition, Crop Protection Publications, ed. C. Tomlin, Bath, United Kingdom and Crop Protection Reference 11^{th} edition, CPR Press, New York, NY, USA, 1995. Plants and descriptions of plants that express *Bacillus thuringiensis* delta endotoxin are available from the Monsanto® and Novartis® agrochemical companies. Plants and description of plants that express genes imparting tolerance to an EPSPS inhibitor herbicide are available from the Monsanto® agrochemical company. Salts of glyphosate are available from the Monsanto® and Zeneca® agrochemical companies.

The following examples illustrate the invention.

### EXAMPLE 1:

Seeds of NuCotn® 32B are planted in a field. Aldicarb, in the form of the commercial product Temik® 15G (a 15% weight/weight granular formulation) is applied in the furrow that comprises the seed at a rate of about 0.5 kg/ha of active ingredient or 3.3 kg/ha of commercial product. At various points in the growing season, the plant tissues are tested for the levels of the delta-endotoxin. The tissues from the plants treated by aldicarb display a higher level of the endotoxin than the tissues from plants not treated by aldicarb.

### EXAMPLE 2:

Seeds of NuCotn® 32B are planted in a field. Aldicarb, in the form of the commercial product Temik® 15G (a 15% weight/weight granular formulation) is applied as a side-dress at 7.8 kg/ha of commercial product. After a full growing season, there are a larger number of bolls on the plants that have been treated with aldicarb in comparison to those not treated with aldicarb. The amount of damage due to bollworm attack on the cotton is less in those treated with aldicarb in-comparison to those not treated with aldicarb.

### EXAMPLE 3:

Cotton leaves and squares were collected from treatments of Bt Cotton wherein the cotton was treated with Temik®15G under the following conditions;
A) 0.45 lbs active ingredient per acre at plant (0.5 kg/ha)
B) 0.75lbs active ingredient per acre at plant; (0.5 kg/ha) and
C) 0.75 lbs active ingredient per acre (0.84 kg/ha) at plant and followed 3 weeks later by a side-dress treatment of 1.0 lbs per acre (1.12 kg/ha) of active ingredient.

One planting was not treated with aldicarb.

After 90 days leaves and squares were collected, washed with soap and water, allowed to dry, washed with acetone, allowed to dry and finally washed with chloroform. The tissues were then freeze dried, ground with a mortar and pestle and stored at room temperature.

The cotton tissues were incorporated into a Heliothis diet and fed to Heliothis virescens. The larvae in the cups were weighed at seven days after infestation and the number of pupae were counted 14 and 21 days after infestation. The percent pupation was calculated at the different concentrations and the EC_{50P} (the amount of tissue'per liter of diet that reduced pupation by 50%) was calculated for each insecticide treatment to express the amount of toxin activity in the treatment.

At the reading two weeks after infestation, the diet containing leaf tissue of cotton plants treated with aldicarb at plant required higher amounts of tissue to reduce pupation by 50% compared to the untreated check, but the diet containing square tissue required lesser amounts of tissue to reduce pupation by 50%.

At the reading three weeks after infestation, the diet containing leaf tissue of cotton plants treated with aldicarb at plant required higher amounts of tissue to reduce pupation by 50% compared to the untreated check, but the diet containing square tissue required lesser amounts of tissue to reduce pupation by 50%.

This indicates that the Bt endotoxin is at least preferably accumulating more in the squares of the Bt cotton plant when treated with aldicarb.

### EXAMPLE 4:

V2 INGARD Cotton was treated with a Temik®15G at plant at a rate of 5 kg per hectare of commercial product. After 3 weeks leaves from the 3^{rd} node were picked and fed to Heliothis armigera 1^{st} instar larvae. At this time the cotton leaves from the third node in the treated plants were less effective than the leaves from the untreated plants.

Two weeks later leaves from the 3^{rd} node and 7^{th} node were taken from treated and untreated plants. Squares were also sampled. When fed to Heliothis armigera larvae, there was a higher mortality in the Temik-treated plant tissue than for the untreated plant tissue. Furthermore, the surviving larvae were significantly smaller when fed the treated leaves than the untreated leaves.

### EXAMPLE 5

A plot of Roundup Ready® Cotton (RRC)was grown up. One part of the plot was treated with Temik® at a rate of 5.6 kg/ha of commercial product at the time of planting of the seed. Another part was treated with Disyston® (disulfoton) at 22.24 kg of commercial product/ha the time of planting of the seed. Another part of the plot was grown from seed treated with Gaucho® (imidacloprid) at a rate of 0.357 kg of commercial product per 100 kg of seed. One part of the plot was left untreated by an insecticide which is called hereafter the untreated check (UTC).

The isopropylamine salt of glyphosate was applied to all the cotton plants at the four-leaf stage in the form of the commercial product Roundup® at a rate of 32 oz of commercial product per acre (= 0.17 kg/ha of active ingredient). The following results were observed 45 days after the Roundup® treatment:

| **Treatment** | **Squares per Plant** | **% change from UTC** |
|---|---|---|
| RRC (UTC) | 7.45 | ---- |
| RRC + aldicarb | 9.05 | +21 |
| RRC + disulfoton | 7.65 | +2 |
| RRC + imidacloprid | 8.70 | +18 |

### EXAMPLE 6

Roundup Ready® Cotton was grown up as in Example 5 and treated with Roundup at the six-leaf stage.

The following results were observed 40 days after the Roundup® treatment:

| **Treatment** | **Squares per Plant** | **% change from UTC** |
|---|---|---|
| RRC (UTC) | 8.85 | ----- |
| RRC + aldicarb | 10.95 | +23% |
| RRC + disulfoton | 5.65 | -36% |
| RRC + imidacloprid | 7.60 | -14% |

The observations of Examples 5 and 6 were made on the same day.

The results of Examples 5 and 6 show that the treatment with aldicarb not only increases the yield of cotton compared with UTC or cotton treated with other herbicides, but also that the time during which glyphosate may be applied without damage to the plant is increased after the four leaf stage, with the same yield improvement, when treatment with other herbicides leads to a decrease of yields.

## Claims

1. A method of increasing foreign protein expression encoded by a foreign gene in plants comprising treating the plant or a seed from which it grows with aldicarb.

2. The method according to claim 1, wherein the plant is selected among the group consisting of cotton, corn, potato, soybean, sunflower, rape seed oil, sugar cane, sugar beet, rice, alfalfa, and banana plants, preferably cotton, corn or potato plants, most preferably a cotton plant.

3. The method according to one of claims 1 or 2, wherein the foreign genes are foreign or heterologous genes coding for proteins of interest to impart herbicide tolerance , disease resistance, toxicity to insects (insect resistance), or to improve quality of the crops.

4. The method according to claim 3, wherein the heterologous or foreign gene encodes a protein to impart herbicide tolerance.

5. The method according to claim 4, wherein heterologous or foreign gene encodes a protein to impart tolerance to an herbicide selected among the group consisiting of oxynil herbicide, EPSPS inhibitor herbicides, including glyphosate and its various salts, glufosinate and HPPD inhibitor herbicides

6. The method according to claim 5, wherein the heterologous or foreign gene encodes a protein to impart tolerance to EPSPS inhibitor herbicides.

7. The method according to claim 3, wherein the heterologous or foreign gene encodes a protein to impart insect resistance.

8. The method according to claim 7, wherein the heterologous or foreign gene encodes for *Bacillus thuringiensis* (*Bt*) endotoxin.

9. The method according to claim 8, wherein the heterologous or foreign gene is selected among the group consisting of *cryI, cryII, cryIII,* and *cryIV* genes.

10. The method according to claim 9, wherein the heterologous or foreign gene is selected among the group consisting of: *cryIA(a); cryIA(b); cryIA(c);* and *cryIIIA(a)* genes.

11. The method according to one of claims 1 to 10, wherein the promoter controlling the expression of the heterologous or foreign gene is selected among the group consisting in the ribulose-biscarboxylase/oxygenase small subunit promoters (SSU RuBisCO) from genes of a variety of species , the histone promoters, the actin promoters, the maize ubiquitin 1 promoters and the 35S promoters of the cauliflower mosaic virus (CaMV 35S).

12. The method according to claim 11, wherein the promoter is the CaMV 35 S promoter.

13. The method according to one of claims 1 to 12, wherein aldicarb is applied directly to the plant and/or to the seed and/or to a locus where the plant is growing or is to be grown.

14. The method as claimed in claim 13, wherein aldicarb is applied to the seed in a seed treatment step before or just after planting, simultaneously or sequentially.

15. The method according to claim 13, wherein aldicarb is applied to the locus before or after planting.

16. The method as claimed in claim 15, wherein aldicarb is applied at the locus after planting but before emergence of the plant.

17. The method as claimed in one of claims 1 to 16, wherein the seeds are treated after planting with aldicarb at a rate of from 0.01 to 6 kg/ha (kilograms of active ingredient per hectare) preferably from 0.05 to 4 kg/ha more preferably from 0.1 to 1 kg/ha.

18. The method as claimed in one of claims 1 to 14, wherein the seeds after treatment with aldicarb before planting comprise from 0.001 % w/w to 20% w/w of aldicarb to seed, preferably from 0.05% to 10%, more preferably from 0.1% to 5%.

19. A method of controlling pests at a locus, which locus comprises a plant containing a gene which encodes for *Bt* endotoxin, the method comprising the application of aldicarb to the plant or to the seed from which it grows.

20. The method according to claim 19, wherein the pest controlled is from the Orders Lepidoptera, Coleoptera or Diptera.

21. The method according to one of claims 19 to 20, wherein plant is selected among the group consisitng of cotton, corn, potato, sugar cane and rice plants, preferably cotton, corn and potato plants, most preferably a cotton plant.

22. The method according to claim one of claims 19 to 21, wherein the heterologous or foreign gene is selected among the group consisting of *cryI, cryII, cryIII,* and *cryIV* genes.

23. The method according to claim 22, wherein the heterologous or foreign gene is selected among the group consisting of: *cryIA(a); cryIA(b); cryIA(c);* and *cryIIIA(a)* genes.

24. The method according to one of claims 19 to 23, wherein the promoter controlling the expression of the heterologous or foreign gene is selected among the group consisting in the ribulose-biscarboxylase/oxygenase small subunit promoters (SSU RuBisCO) from genes of a variety of species , the histone promoters, the actin promoters, the maize ubiquitin 1 promoters and the 35S promoters of the cauliflower mosaic virus (CaMV 35S).

25. The method according to claim 24, wherein the promoter is the CaMV 35 S promoter.

26. The method according to one of claims 19 to 25, wherein aldicarb is applied directly to the plant and/or to the seed and/or to a locus where the plant is growing or is to be grown.

27. The method as claimed in claim 26, wherein aldicarb is applied to the seed in a seed treatment step before or just after planting, simultaneously or sequentially.

28. The method according to claim 26, wherein aldicarb is applied to the locus before or after planting.

29. The method as claimed in claim 28, wherein aldicarb is applied at the locus after planting but before emergence of the plant.

30. The method as claimed in one of claims 19 to 29, wherein the seeds are treated after planting with aldicarb at a rate of from 0.01 to 6 kg/ha (kilograms of active ingredient per hectare) preferably from 0.05 to 4 kg/ha more preferably from 0.1 to 1 kg/ha.

31. The method as claimed in one of claims 19 to 28, wherein the seeds after treatment with aldicarb before planting comprise from 0.001 % w/w to 20% w/w of aldicarb to seed, preferably from 0.05% to 10%, more preferably from 0.1% to 5%.

32. A method of controlling weeds at a locus, which locus comprises a plant containing a foreign gene which encodes a protein imparting tolerance to an herbicide, wherein the said herbicide is applied in said locus to said plant, the method comprising the application of aldicarb to the plant or to the seed from which it grows.

33. A method of increasing the time during which an herbicide may be applied to an herbicide tolerant plant, which method comprises applying aldicarb to the said plant or to a seed from which it grows.

34. The method as claimed in one of claims 32 or 33, wherein the herbicide tolerant plant is selected among cotton, corn, soybean, sunflower, rape seed oil, sugar cane, sugar beet or alfalfa plants, preferably cotton, corn or soybean plants, most preferably a cotton plant.

35. The method as claimed in one of claims 32 to 34, wherein the herbicide is an EPSPS inhibitor herbicide, including glyphosate and its various salts, like the isopropylammonium salt or the sulfosate salt, and the foreign gene encodes for a protein imparting tolerance to the said EPSPS inhibitor herbicide.

36. The method according to one of claims 32 to 35, wherein the promoter controlling the expression of the heterologous or foreign gene is selected among the group consisting in the ribulose-biscarboxylase/oxygenase small subunit promoters (SSU RuBisCO) from genes of a variety of species , the histone promoters, the actin promoters, the maize ubiquitin 1 promoters and the 35S promoters of the cauliflower mosaic virus (CaMV 35S).

37. The method according to claim 36, wherein the promoter is the CaMV 35 S promoter.

38. The method according to one of claims 32 to 37, wherein aldicarb is applied directly to the plant and/or to the seed and/or to a locus where the plant is growing or is to be grown.

39. The method according to claim 38, wherein aldicarb is applied to the seed in a seed treatment step before or just after planting, simultaneously or sequentially.

40. The method according to claim 38, wherein aldicarb is applied to the locus before or after planting.

41. The method as claimed in claim 40, wherein aldicarb is applied at the locus after planting but before emergence of the plant.

42. The method as claimed in one of claims 32 to 41, wherein the seeds are treated after planting with aldicarb at a rate of from 0.01 to 6 kg/ha (kilograms of active ingredient per hectare) preferably from 0.05 to 4 kg/ha more preferably from 0.1 to 1 kg/ha.

43. The method as claimed in one of claims 32 to 39, wherein the seeds after treatment with aldicarb before planting comprise from 0.001 % w/w to 20% w/w of aldicarb to seed, preferably from 0.05% to 10%, more preferably from 0.1% to 5%.

44. A new composition which comprises aldicarb and a seed containing a foreign gene which encodes for a foreign protein.

45. The composition according to claim 44, wherein it comprises from 0.001 % w/w to 20% w/w of aldicarb to seed, preferably from 0.05% to 10%, more preferably from 0.1% to 5%.

46. A plant which comprises a foreign gene which encodes for a foreign protein as disclosed in one of the preceding claims, wherein the said plant has an improved amount of foreign protein.

## Patentansprüche

1. Verfahren zur Erhöhung der fremdgencodierten Fremdproteinexpression in Pflanzen, **dadurch gekennzeichnet, daß** man die Pflanze oder Saatgut, aus dem sie heranwächst, mit Aldicarb behandelt.

2. Verfahren nach Anspruch 1, wobei die Pflanze aus der Gruppe Baumwoll-, Mais-, Kartoffel-, Sojabohnen-, Sonnenblumen-, Raps-, Zuckerrohr-, Zuckerrüben-, Reis-, Luzerne- und Bananenpflanzen, vorzugsweise Baumwoll-, Mais- oder Kartoffelpflanzen, stammt und am stärksten bevorzugt eine Baumwollpflanze ist.

3. Verfahren nach Anspruch 1 oder 2, wobei es sich bei den Fremdgenen um Fremdgene oder heterologe Gene handelt, die für interessierende Proteine zur Vermittlung von Herbizidtoleranz, Krankheitsresistenz, Insektentoxizität (Insektenresistenz) oder zur Verbesserung der Qualität des Ernteguts codieren.

4. Verfahren nach Anspruch 3, wobei das heterologe Protein oder Fremdprotein für ein Protein codiert, das Herbizidtoleranz vermittelt.

5. Verfahren nach Anspruch 4, wobei das heterologe Protein oder Fremdprotein für ein Protein codiert, das eine Toleranz gegenüber einem Herbizid aus der Gruppe Oxynilherbizid, EPSPS-Hemmstoff-Herbizide, darunter Glyphosate und seine verschiedenen Salze, Glufosinate und HPPD-Hemmstoff-Herbizide vermittelt.

6. Verfahren nach Anspruch 5, wobei das heterologe Gen oder Fremdgen für ein Protein codiert, das Toleranz gegenüber EPSPS-Hemmstoff-Herbiziden vermittelt.

7. Verfahren nach Anspruch 3, wobei das heterologe Gen oder Fremdgen für ein Protein codiert, das Insektenresistenz vermittelt.

8. Verfahren nach Anspruch 7, wobei das heterologe Gen oder Fremdgen für *Bacillus thuringiensis (Bt)*-Endotoxin codiert.

9. Verfahren nach Anspruch 8, wobei das heterologe Gen oder Fremdgen aus der Gruppe der *cryI-, cryII-, cry-III-* und *cryIV*-Gene stammt.

10. Verfahren nach Anspruch 9, wobei das heterologe Gen oder Fremdgen aus der Gruppe der *cryIA(a)-, cryIA(b)-, cryIA(c)- und cryIIIA(a)*-Gene stammt.

11. Verfahren nach einem der Ansprüche 1 bis 10, wobei der die Expression des heterologen Gens oder Fremdgens kontrollierende Promoter aus der Gruppe der Promoter der kleinen Untereinheit der Ribulosebiscarboxylase/Oxygenase (SSU RuBisCO) von Genen verschiedener Arten, der HistonPromoter, der ActinPromoter, der Ubiquitin-1-Promoter aus Mais und der 35S-Promoter des Blumenkohlmosaikvirus (CaMV 35S) stammt.

12. Verfahren nach Anspruch 11, wobei es sich bei dem Promoter um den CaMV-35S-Promoter handelt.

13. Verfahren nach einem der Ansprüche 1 bis 12, wobei Aldicarb direkt auf die Pflanze und/oder das Saatgut und/oder einen Standort, auf dem die Pflanze wächst oder wachsen soll, ausgebracht wird.

14. Verfahren nach Anspruch 13, wobei Aldicarb direkt auf das Saatgut in einem Saatgutbehandlungsschritt vor oder unmittelbar nach dem Pflanzen zugleich oder der Reihe nach ausgebracht wird.

15. Verfahren nach Anspruch 13, wobei Aldicarb auf den Standort vor oder nach dem Pflanzen ausgebracht wird.

16. Verfahren nach Anspruch 15, wobei Aldicarb auf den Standort nach dem Pflanzen, jedoch vor dem Auflaufen der Pflanze ausgebracht wird.

17. Verfahren nach einem der Ansprüche 1 bis 16, wobei die Samen nach dem Pflanzen mit Aldicarb in einer Aufwandmenge von 0,01 bis 6 kg/ha (Kilogramm Wirkstoff pro Hektar), vorzugsweise 0,05 bis 4 kg/ha, stärker bevorzugt 0,1 bis 1 kg/ha, behandelt werden.

18. Verfahren nach einem der Ansprüche 1 bis 14, wobei die Samen nach der Behandlung mit Aldicarb vor dem Pflanzen 0,001% (w/w) bis 20% (w/w), bevorzugt 0,05% bis 10%, stärker bevorzugt 0,1% bis 5%, Aldicarb in bezug auf das Saatgut enthalten.

19. Verfahren zur Bekämpfung von Schädlingen an einem Standort, wobei der Standort eine Pflanze mit einem Gen, das für *Bt*-Endotoxin codiert, enthält, wobei bei dem Verfahren Aldicarb auf die Pflanze oder das Saatgut, aus dem sie heranwächst, ausgebracht wird.

20. Verfahren nach Anspruch 19, wobei der bekämpfte Schädling aus den Ordnungen Lepidoptera, Coleoptera oder Diptera stammt.

21. Verfahren nach Anspruch 19 oder 20, wobei die Pflanze aus der Gruppe Baumwoll-, Mais-, Kartoffel-, Zuckerrohr- und Reispflanzen, vorzugsweise Baumwoll-, Mais- und Kartoffelpflanzen, stammt und am stärksten bevorzugt eine Baumwollpflanze ist.

22. Verfahren nach einem der Ansprüche 19 bis 21, wobei das heterologe Gen oder Fremdgen aus der Gruppe der *cryI-, cryII-, cryIII- und cryIV*-Gene stammt.

23. Verfahren nach Anspruch 22, wobei das heterologe Gen oder Fremdgen aus der Gruppe der *cryIA(a)-, cryIA(b)-*, *cryIA(c)- und cryIIIA(a)*-Gene stammt.

24. Verfahren nach einem der Ansprüche 19 bis 23, wobei der die Expression des heterologen Gens oder Fremdgens kontrollierende Promoter aus der Gruppe der Promoteren der kleinen Untereinheit der Ribulosebiscarboxylase/Oxygenase (SSU RuBisCO) von Genen verschiedener Arten, der HistonPromoteren, der ActinPromoteren, der Ubiquitin-1-Promoteren aus Mais und der 35S-Promoteren des Blumenkohlmosaikvirus (CaMV 35S) stammt.

25. Verfahren nach Anspruch 24, wobei es sich bei dem Promoter um den CaMV-35S-Promoter handelt.

26. Verfahren nach einem der Ansprüche 19 bis 25, wobei Aldicarb direkt auf die Pflanze und/oder das Saatgut und/oder einen Standort, auf dem die Pflanze wächst oder wachsen soll, ausgebracht wird.

27. Verfahren nach Anspruch 26, wobei Aldicarb direkt auf das Saatgut in einem Saatgutbehandlungsschritt vor oder unmittelbar nach dem Pflanzen zugleich oder der Reihe nach ausgebracht wird.

28. Verfahren nach Anspruch 26, wobei Aldicarb auf den Standort vor oder nach dem Pflanzen ausgebracht wird.

29. Verfahren nach Anspruch 28, wobei Aldicarb auf den Standort nach dem Pflanzen, jedoch vor dem Auflaufen der Pflanze ausgebracht wird.

30. Verfahren nach einem der Ansprüche 19 bis 29, wobei die Samen nach dem Pflanzen mit Aldicarb in einer Aufwandmenge von 0,01 bis 6 kg/ha (Kilogramm Wirkstoff pro Hektar), vorzugsweise 0,05 bis 4 kg/ha, stärker bevorzugt 0,1 bis 1 kg/ha, behandelt werden.

31. Verfahren nach einem der Ansprüche 19 bis 28, wobei die Samen nach der Behandlung mit Aldicarb vor dem Pflanzen 0,001% (w/w) bis 20% (w/w), bevorzugt 0,05% bis 10%, stärker bevorzugt 0,1% bis 5%, Aldicarb in bezug auf das Saatgut enthalten.

32. Verfahren zur Bekämpfung von Unkräutern auf einem Standort, wobei dieser Standort eine Pflanze mit einem Fremdgen enthält, das für ein Protein codiert, das Toleranz gegenüber einem Herbizid vermittelt, wobei das Herbizid an diesem Standort auf diese Pflanze ausgebracht wird, wobei bei dem Verfahren Aldicarb auf die Pflanze oder das Saatgut, aus dem sie heranwächst, ausgebracht wird.

33. Verfahren zur Verlängerung des Zeitraums während dessen ein Herbizid auf eine herbizidtolerante Pflanze ausgebracht werden kann, wobei bei dem Verfahren Aldicarb auf die Pflanze oder das Saatgut, aus dem sie heranwächst, ausgebracht wird.

34. Verfahren nach Anspruch 32 oder 33, wobei die herbizidtolerante Pflanze aus der Gruppe Baumwoll-, Mais-, Sojabohnen-, Sonnenblumen-, Raps-, Zuckerrohr-, Zuckerrüben- oder Luzernepflanzen, vorzugsweise Baumwoll-, Mais- oder Sojabohnenpflanzen, stammt und am stärksten bevorzugt eine Baumwollpflanze ist.

35. Verfahren nach einem der Ansprüche 32 bis 34, wobei es sich bei dem Herbizid um ein EPSPS-Hemmstoff-Herbizid, darunter auch Glyphosat und seine verschiedenen Salze, wie das Isopropylammoniumsalz oder das Sulfosatsalz, handelt und das Fremdgen für ein Protein codiert, das Toleranz gegenüber dem EPSPS-Hemmstoff-Herbizid vermittelt.

36. Verfahren nach einem der Ansprüche 32 bis 35, wobei der die Expression des heterologen Gens oder Fremdgens kontrollierende Promoter aus der Gruppe der Promoteren der kleinen Untereinheit der Ribulosebiscarboxylase/Oxygenase (SSU RuBisCO) von Genen verschiedener Arten, der HistonPromoteren, der ActinPromoteren, der Ubiquitin-1-Promoteren aus Mais und der 35S-Promoteren des Blumenkohlmosaikvirus (CaMV 35S) stammt.

37. Verfahren nach Anspruch 36, wobei es sich bei dem Promoter um den CaMV-35S-Promoter handelt.

38. Verfahren nach einem der Ansprüche 32 bis 37, wobei Aldicarb direkt auf die Pflanze und/oder das Saatgut und/oder einen Standort, auf dem die Pflanze wächst oder wachsen soll, ausgebracht wird.

39. Verfahren nach Anspruch 38, wobei Aldicarb direkt auf das Saatgut in einem Saatgutbehandlungsschritt vor oder unmittelbar nach dem Pflanzen zugleich oder der Reihe nach ausgebracht wird.

40. Verfahren nach Anspruch 38, wobei Aldicarb auf den Standort vor oder nach dem Pflanzen ausgebracht wird.

41. Verfahren nach Anspruch 40, wobei Aldicarb auf den Standort nach dem Pflanzen, jedoch vor dem Auflaufen der Pflanze ausgebracht wird.

42. Verfahren nach einem der Ansprüche 32 bis 41, wobei die Samen nach dem Pflanzen mit Aldicarb in einer Aufwandmenge von 0,01 bis 6 kg/ha (Kilogramm Wirkstoff pro Hektar), vorzugsweise 0,05 bis 4 kg/ha, stärker bevorzugt 0,1 bis 1 kg/ha, behandelt werden.

43. Verfahren nach einem der Ansprüche 32 bis 39, wobei die Samen nach der Behandlung mit Aldicarb vor dem Pflanzen 0,001% (w/w) bis 20% (w/w), bevorzugt 0,05% bis 10%, stärker bevorzugt 0,1% bis 5%, Aldicarb in bezug auf das Saatgut enthalten.

44. Neues Produkt, enthaltend Aldicarb und Saatgut, das ein für ein Fremdprotein codierendes Fremdgen enthält.

45. Produkt nach Anspruch 44, das 0,001 % (w/w) bis 20% (w/w), vorzugsweise 0,05% bis 10%, stärker bevorzugt 0,1% bis 5% Aldicarb in bezug auf das Saatgut enthält.

46. Pflanze enthaltend ein für ein Fremdprotein codierendes Fremdgen nach einem der vorhergehenden Ansprüche, wobei die Pflanze eine erhöhte Menge an Fremdprotein enthält.

## Revendications

1. Méthode d'augmentation de l'expression d'une protéine étrangère codée par un gène étranger dans des plantes, comprenant le traitement de la plante ou d'une graine dont elle est issue par l'aldicarbe.

2. Méthode selon la revendication 1, dans laquelle la plante est choisie parmi le groupe constitué par le coton, le blé, la pomme de terre, le soja, le tournesol, le colza, la canne à sucre, la betterave à sucre, le riz, la luzerne et le bananier, préférablement le coton, le blé ou la pomme de terre, tout préférablement le coton.

3. Méthode selon l'une des revendications 1 ou 2, dans laquelle les gènes étrangers sont des gènes étrangers ou hétérologues codant pour des protéines d'intérêt pour conférer une tolérance aux herbicides, une résistance aux maladies, une toxicité pour les insectes (résistance aux insectes) ou pour améliorer la qualité des cultures.

4. Méthode selon la revendication 3, dans laquelle le gène hétérologue ou étranger code pour une protéine pour conférer une tolérance aux herbicides.

5. Méthode selon la revendication 4, dans laquelle le gène hétérologue ou étranger code pour une protéine pour conférer une tolérance à un herbicide choisi parmi le groupe constitué par un herbicide de type oxynil, les herbicides à inhibition de la EPSPS, y compris le glyphosate et ses divers sels, le glufosinate et les herbicides à inhibition de la HPPD.

6. Méthode selon la revendication 5, dans laquelle le gène hétérologue ou étranger code pour une protéine pour conférer une tolérance aux herbicides à inhibition de la EPSPS.

7. Méthode selon la revendication 3, dans laquelle le gène hétérologue ou étranger code pour une protéine pour conférer une résistance aux insectes.

8. Méthode selon la revendication 7, dans laquelle le gène hétérologue ou étranger code pour une endotoxine de *Bacillus thuringiensis (Bt).*

9. Méthode selon la revendication 8, dans laquelle le gène hétérologue ou étranger est choisi parmi le groupe constitué par les gènes *cryI, cryII, cryIII* et *cryIV.*

10. Méthode selon la revendication 9, dans laquelle le gène hétérologue ou étranger est choisi parmi le groupe constitué par les gènes *cryIA(a), cryIA(b)*, *cryIA(c)* et *cryIIIA(a).*

11. Méthode selon l'une des revendications 1 à 10, dans laquelle le promoteur régulant l'expression du gène hétérologue ou étranger est choisi parmi le groupe constitué par les promoteurs de la petite sous-unité de la ribulose-biscarboxylase/oxygénase (SSU RuBisCO) issus de gènes de diverses espèces, les promoteurs d'histone, les promoteurs d'actine, les promoteurs de l'ubiquitine I du maïs et les promoteurs 35S du virus de la mosaïque du chou-fleur (CaMV 35S).

12. Méthode selon la revendication 11, dans laquelle le promoteur est le promoteur CaMV 35S.

13. Méthode selon l'une des revendications 1 à 12, dans laquelle l'aldicarbe est appliqué directement à la plante et/ou à la graine et/ou à un lieu où la plante pousse ou doit pousser.

14. Méthode selon la revendication 13, dans laquelle l'aldicarbe est appliqué à la graine dans une étape de traitement de graines avant ou juste après la plantation, de manière simultanée ou séquentielle.

15. Méthode selon la revendication 13, dans laquelle l'aldicarbe est appliqué au lieu avant ou après la plantation.

16. Méthode selon la revendication 15, dans laquelle l'aldicarbe est appliqué au lieu après la plantation, mais avant l'émergence de la plante.

17. Méthode selon l'une des revendications 1 à 16, dans laquelle les graines sont traitées après la plantation par l'aldicarbe, à une dose allant de 0,01 à 6 kg/ha (kilogrammes d'ingrédient actif par hectare), préférablement de 0,05 à 4 kg/ha, plus préférablement de 0,1 à 1 kg/ha.

18. Méthode selon l'une des revendications 1 à 14, dans laquelle les graines après un traitement par l'aldicarbe et avant la plantation contiennent de 0,001% p/p à 20% p/p d'aldicarbe par rapport aux graines, préférablement de 0,05% à 10%, plus préférablement de 0,1% à 5%.

19. Méthode de lutte contre des insectes nuisibles en un lieu, lequel lieu comprend une plante contenant un gène codant pour une endotoxine de *Bt*, la méthode comprenant l'application d'aldicarbe à la plante ou à la graine dont elle est issue.

20. Méthode selon la revendication 19, dans laquelle l'insecte nuisible contrôlé appartient à l'ordre des Lépidoptères, des Coléoptères ou des Diptères.

21. Méthode selon l'une des revendications 19 à 20, dans laquelle la plante est choisie parmi le groupe constitué par le coton, le blé, la pomme de terre, la canne à sucre et le riz, préférablement le coton, le blé et la pomme de terre, tout préférablement le coton.

22. Méthode selon l'une des revendications 19 à 21, dans laquelle le gène hétérologue ou étranger est choisi parmi le groupe constitué par les gènes *cryI, cryII, cryIII* et *cryIV.*

23. Méthode selon la revendication 22, dans laquelle le gène hétérologue ou étranger est choisi parmi le groupe constitué par les gènes *cryIA(a), cryIA(b), cryIA(c)* et *cryIIIA(a).*

24. Méthode selon l'une des revendications 19 à 23, dans laquelle le promoteur régulant l'expression du gène hétérologue ou étranger est choisi parmi le groupe constitué par les promoteurs de la petite sous-unité de la ribulose-biscarboxylase/oxygénase (SSU RuBisCO) issus de gènes de diverses espèces, les promoteurs d'histone, les promoteurs d'actine, les promoteurs de l'ubiquitine I du maïs et les promoteurs 35S du virus de la mosaïque du chou-fleur (CaMV 35S).

25. Méthode selon la revendication 24, dans laquelle le promoteur est le promoteur CaMV 35S.

26. Méthode selon l'une des revendications 19 à 25, dans laquelle l'aldicarbe est appliqué directement à la plante et/ou à la graine et/ou à un lieu où la plante pousse ou doit pousser.

27. Méthode selon la revendication 26, dans laquelle l'aldicarbe est appliqué à la graine dans une étape de traitement de graines avant ou juste après la plantation, de manière simultanée ou séquentielle.

28. Méthode selon la revendication 26, dans laquelle l'aldicarbe est appliqué au lieu avant ou après la plantation.

29. Méthode selon la revendication 28, dans laquelle l'aldicarbe est appliqué au lieu après la plantation, mais avant l'émergence de la plante.

30. Méthode selon l'une des revendications 19 à 29, dans laquelle les graines sont traitées après la plantation par l'aldicarbe, à une dose allant de 0,01 à 6 kg/ha (kilogrammes d'ingrédient actif par hectare), préférablement de 0,05 à 4 kg/ha, plus préférablement de 0,1 à 1 kg/ha.

31. Méthode selon l'une des revendications 19 à 28, dans laquelle les graines après un traitement par l'aldicarbe et avant la plantation contiennent de 0,001% p/p à 20% p/p d'aldicarbe par rapport aux graines, préférablement de 0,05% à 10%, plus préférablement de 0,1% à 5%.

32. Méthode de lutte contre des mauvaises herbes en un lieu, lequel lieu comprend une plante contenant un gène étranger codant pour une protéine conférant une tolérance à un herbicide, dans laquelle ledit herbicide est appliqué audit lieu à ladite plante, la méthode comprenant l'application d'aldicarbe à la plante ou à la graine dont elle est issue.

33. Méthode d'augmentation de l'intervalle pendant lequel un herbicide peut être appliqué à une plante tolérante aux herbicides, laquelle méthode comprend l'application d'aldicarbe à ladite plante ou à une graine dont elle est issue.

34. Méthode selon l'une des revendications 32 ou 33, dans laquelle la plante tolérante aux herbicides est choisie parmi le coton, le blé, le soja, le tournesol, le colza, la canne à sucre, la betterave à sucre ou la luzerne, préférablement le coton, le blé ou le soja, tout préférablement le coton.

35. Méthode selon l'une des revendications 32 à 34, dans laquelle l'herbicide est un herbicide à inhibition de la EPSPS, y compris le glyphosate et ses divers sels, comme le sel d'isopropylammonium ou le sel de sulfosate, et le gène étranger code pour une protéine pour conférer une tolérance audit herbicide à inhibition de la EPSPS.

36. Méthode selon l'une des revendications 32 à 35, dans laquelle le promoteur régulant l'expression du gène hétérologue ou étranger est choisi parmi le groupe constitué par les promoteurs de la petite sous-unité de la ribulose-biscarboxylase/oxygénase (SSU RuBisCO) issus de gènes de diverses espèces, les promoteurs d'histone, les promoteurs d'actine, les promoteurs de l'ubiquitine I du maïs et les promoteurs 35S du virus de la mosaïque du chou-fleur (CaMV 35S).

37. Méthode selon la revendication 36, dans laquelle le promoteur est le promoteur CaMV 35S.

38. Méthode selon l'une des revendications 32 à 37, dans laquelle l'aldicarbe est appliqué directement à la plante et/ou à la graine et/ou à un lieu où la plante pousse ou doit pousser.

39. Méthode selon la revendication 38, dans laquelle l'aldicarbe est appliqué à la graine dans une étape de traitement de graines avant ou juste après la plantation, de manière simultanée ou séquentielle.

40. Méthode selon la revendication 38, dans laquelle l'aldicarbe est appliqué au lieu avant ou après la plantation.

41. Méthode selon la revendication 40, dans laquelle l'aldicarbe est appliqué au lieu après la plantation, mais avant l'émergence de la plante.

42. Méthode selon l'une des revendications 32 à 41, dans laquelle les graines sont traitées après la plantation par l'aldicarbe, à une dose allant de 0,01 à 6 kg/ha (kilogrammes d'ingrédient actif par hectare), préférablement de 0,05 à 4 kg/ha, plus préférablement de 0,1 à 1 kg/ha.

43. Méthode selon l'une des revendications 32 à 39, dans laquelle les graines après un traitement par l'aldicarbe et avant la plantation contiennent de 0,001% p/p à 20% p/p d'aldicarbe par rapport aux graines, préférablement de 0,05% à 10%, plus préférablement de 0,1% à 5%.

44. Nouvelle composition comprenant de l'aldicarbe et une graine contenant un gène étranger codant pour une protéine étrangère.

45. Composition selon la revendication 44, dans laquelle elle comprend de 0,001% p/p à 20% p/p d'aldicarbe par rapport aux graines, préférablement de 0,05% à 10%, plus préférablement de 0,1% à 5%.

46. Plante comprenant un gène étranger codant pour une protéine étrangère tel que divulgué selon l'une des revendications précédentes, dans laquelle ladite plante possède une quantité améliorée de protéine étrangère.
